# EUROPÄISCHE PATENTSCHRIFT

(11) **EP 1 611 858 B1**
(45) Veröffentlichungstag und Bekanntmachung des Hinweises auf die Patenterteilung: **06.04.2011**
(21) Anmeldenummer: 05001771.4
(22) Anmeldetag: 28.01.2005
(51) Int. Cl.: A61B 18/14

(54) **Medizinisches Instrument für die Elektrochirurgie**
Medical instrument for electrosurgery
Instrument médical pour électrochirurgie

(30) Priorität: 02.07.2004 DE 202004010780 U
(43) Veröffentlichungstag der Anmeldung: 04.01.2006
(73) Patentinhaber: Karl Storz GmbH & Co. KG, 78532 Tuttlingen (DE)
(72) Erfinder: Anders, Fridolin, 78194 Immedingen-Zimmern (DE); Brückler, Hubert, 78532 Tuttlingen (DE)
(74) Vertreter: Heuckeroth, Volker

(56) Entgegenhaltungen:
- US-A1- 2002 128 649
- US-A1- 2004 116 924
- US-B1- 6 334 860
- US-B1- 6 394 998

## Beschreibung

Die Erfindung betrifft ein medizinisches Instrument für die Elektrochirurgie, mit einem Rohrschaft, mit zumindest einem um eine Schwenkachse relativ zum Rohrschaft beweglichen Maulteil am distalen Ende des Rohrschafts, wobei das zumindest eine bewegliche Maulteil eine Elektrode aufweist, und mit einer zu der Elektrode des zumindest einen beweglichen Maulteils führenden Stromzuleitung, die als drahtförmiges Element aus stromleitendem Material ausgebildet ist, dessen distales Ende mit der Elektrode verbunden ist, wobei das drahtförmige Element im Bereich der Schwenkachse als Schleife ausgebildet ist, und wobei das drahtförmige Element zumindest im Bereich der Schleife aus einem federelastischen Material gebildet ist.

Ein derartiges Instrument ist aus dem Dokument US-B1-6 394 998 bekannt.

Ein Instrument der eingangs genannten Art wird bspw. zur Durchführung endoskopisch gestützter Eingriffe im menschlichen oder tierischen Körper im Rahmen der minimal-invasiven Chirurgie verwendet.

Mit einem elektrochirurgischen medizinischen Instrument der vorliegenden Erfindung kann biologisches Gewebe unter dem Einfluss von Hochfrequenzstrom geschnitten und/oder koaguliert werden. Üblicherweise sind am distalen Ende des Rohrschafts zwei Maulteile angeordnet, von denen zumindest eines beweglich ist, wobei die vorliegende Erfindung besonders vorteilhaft bei solchen medizinischen Instrumenten ist, bei denen beide Maulteile beweglich sind. Je nach dem chirurgischen Verwendungszweck eines solchen Instruments sind die Maulteile als Schneidwerkzeuge mit Schneidkanten ausgebildet, um Gewebe im Körper abzutrennen, oder als Fasswerkzeuge mit entsprechend stumpf aufeinander stoßenden Flächen, um abgetrenntes Gewebe mit den Maulteilen zu fassen und aus dem Körper zu entfernen, oder um ein Organ oder ein Gefäß zu halten, um dieses aus dem Operationsgebiet zu verlegen. Die Maulteile können auch eine Kombinaton aus einer Schneid- und einer Fassfunktion aufweisen.

Wenn das Instrument zwei Maulteile aufweist, ist zumindest eines der beiden Maulteile gelenkig mit dem Rohrschaft verbunden, während das andere Maulteil mit dem Rohrschaft starr oder ebenfalls gelenkig verbunden ist.

Bei einem medizinischen Instrument der eingangs genannten Art ist es weiterhin vorgesehen, dass zumindest ein Maulteil eine mit Hochfrequenzstrom beaufschlagbare Elektrode aufweist. Wenn das Instrument als bipolares elektrochirurgisches Instrument ausgebildet ist, was im Rahmen der vorliegenden Erfindung ebenfalls bevorzugt ist, weisen beide Maulteile jeweils eine mit Hochfrequenzstrom beaufschlagbare Elektrode mit unterschiedlichen Polaritäten auf. Beide Elektroden der beiden Maulteile können dementsprechend getrennt voneinander mit jeweils einem Pol einer Hochfrequenzspannungsquelle verbunden werden. Durch Beaufschlagen der Elektroden der beiden Maulteile kann einerseits im Falle der Ausbildung als Schneidwerkzeug die Schneidwirkung durch die thermische Wirkung des Hochfrequenzstroms im Gewebe erhöht werden, andererseits kann im Falle einer Ausbildung als Fasswerkzeug zwischen den Maulteilen gefasstes Gewebe durch die Wärmeentwicklung koaguliert und eine Blutung des Gewebes damit gestillt werden.

Bei derartigen elektrochirurgischen medizinischen Instrumenten stellt sich die Stromzuführung zu der zumindest einen Elektrode des zumindest einen beweglichen Maulteils regelmäßig als problematisch dar. Insbesondere bei bipolaren Instrumenten müssen die beiden Maulteile im Bereich des die Schwenkachse des zumindest einen Maulteils bildenden Gelenks ausreichend elektrisch gegeneinander isoliert sein. Eine elektrische Trennung der beiden Maulteile im Bereich des Gelenks ist insbesondere bei bipolaren Instrumenten erforderlich, da die Elektroden der beiden Maulteile an unterschiedliche Potenziale gelegt werden. Das Problem der elektrischen Trennung der beiden Maulteile voneinander stellt sich als um so größer dar, je kleiner ein derartiges Instrument im Bereich der Maulteile und damit im Bereich des Gelenks ausgebildet ist. Eine schmalbauende Ausgestaltung des Instruments im Bereich der Maulteile ist jedoch für die minimal-invasive Chirurgie besonders wichtig.

Bei einem aus dem Dokument WO-A-00/36986 bekannten Instrument sind am distalen Ende zwei Maulteile vorgesehen, von denen nur eines beweglich ist, wobei beide Maulteile jeweils einen metallischen Grundkörper aufweisen, die im Bereich der Schwenkachse miteinander gelenkig verbunden sind. Die metallischen Grundkörper weisen auf ihrer einander zugewandten Seite jeweils ein Isolatorelement auf, an denen die jeweilige Elektrode des jeweiligen Maulteils angebracht ist, so dass die Elektroden vom metallischen Grundkörper elektrisch getrennt sind. Zu jeder der beiden Elektroden verläuft über das Gelenk hinweg eine Stromzuleitung, wobei die Stromzuleitungen als drahtförmige Elemente ausgebildet sind und voneinander isoliert sind. Bei diesem Aufbau der Maulteile ist es möglich, die Maulteile im Bereich des Gelenks metallisch und damit besonders stabil auszugestalten.

Nachteil dieser Ausgestaltung ist die Art der Stromzuführung zu der jeweiligen Elektrode in Form des gerade verlaufenden, drahtförmigen Elements. Beim Öffnen und Schließen der Maulteille, das heißt beim Bewegen des zumindest einen beweglichen Maulteils, wird das drahtförmige Element permanent gebogen und wieder gestreckt. Diese über die Lebensdauer des Instruments gesehen ständigen Lastwechsel führen relativ rasch zu einem Ermüdungsbruch des drahtförmigen Elements im Bereich der Schwenkachse, wodurch das Instrument keine hohen Standzeiten erreicht, das heißt, die Lebensdauer dieses Instruments ist unerwünscht verkürzt.

Zur Behebung dieses Problems hat man daher für das zumindest eine bewegliche Maulteil einen Schleifkontakt vorgesehen, der den Strom am die Schwenkachse des zumindest einen beweglichen Maulteils bildenden Gelenk abgreift, wobei die Stromzuleitung entsprechend am Gelenk endet. Das Vorsehen eines Schleifkontakts im Bereich des Gelenks ist jedoch konstruktiv sehr aufwändig, und bei besonders kleinbauenden Instrumenten sehr schwierig zu realisieren.

Das aus dem oben genannten Dokument US-B1-6 394 998 bekannte elektrochirurgische Instrument weist am distalen Ende eine Elektrode auf, die über ein elektrisch leitfähiges Kabel mit Spannung versorgt werden kann, das sich von einem Stecker am proximalen Ende zu der Elektrode erstreckt. Das leitfähige Kabel ist in Form einer Schleife um die Schwenkachse der Elektrode gelegt, um beim Verschwenken der Elektrode eine erhöhte mechanische Spannung oder Dehnung im Kabel zu vermeiden.

Aus dem Dokument WO-A-01/15614 ist ein elektrochirurgisches, insbesondere bipolares medizinisches Instrument bekannt, das zwei Maulteile aufweist, von denen eines beweglich ist. Die beiden Maulteile weisen im Bereich ihrer gelenkigen Verbindung einen Grundkörper aus einem elektrisch isolierenden Material auf, an dem ein die zugehörige Elektrode bildender elektrisch leitender Maulteileinsatz befestigt ist. Die Stromzuführung zu der Elektrode des beweglichen Maulteils erfolgt über das zum Öffnen und Schließen der Maulteile vorgesehenes axial bewegliches Kraftübertragungselement, während die Elektrode des unbeweglichen Maulteils über ein gerade verlaufendes, drahtförmiges Element, das proximal mit dem ebenfalls als Stromzuführung dienenden Rohrschaft verbunden ist, und dessen distales Ende mit der Elektrode des unbeweglichen Maulteils verbunden ist.

Würde auch das zweite Maulteil beweglich ausgestaltet, bestünde das gleiche Problem wie bei dem zuvor beschriebenen bekannten Instrument, nämlich dass das drahtförmige Element permanent auf Biegung und Streckung beansprucht würde, was auf Dauer wiederum zu einem Bruch des drahtförmigen Elements führen würde.

Der Erfindung liegt daher die Aufgabe zu Grunde, ein medizinisches Instrument der eingangs genannten Art weiter zu verbessern.

Erfindungsgemäß wird diese Aufgabe hinsichtlich des eingangs genannten medizinischen Instruments dadurch gelöst, dass das drahtförmige Element zumindest im Bereich der Schleife aus Federstahl gebildet ist.

Anstatt wie bei dem bekannten Instrument das drahtförmige Element im Wesentlichen geradlinig an dem die Schwenkachse bildenden Gelenk vorbeizuführen, ist bei dem erfindungsgemäßen medizinischen Instrument vorgesehen, das drahtförmige Element im Bereich der Schwenkachse zu einer Schleife zu legen, die einen Längenausgleich beim Verschwenken des zumindest einen beweglichen Maulteils um die Schwenkachse gewährleistet. Beim Hin- und Herverschwenken des zumindest einen beweglichen Maulteils beim Gebrauch des Instruments wird das drahtförmige Element somit nicht mehr permanent auf Biegung beansprucht, weil die permanente Winkeländerung des zumindest einen beweglichen Maulteils relativ zum Schaft an die Schleife weitergegeben und von dieser in eine Längenzunahme bzw. Längenabnahme am Umfang der Schleife umgewandelt wird. Die Schleife wirkt entsprechend wie eine Art Drehfeder.

Die Schleife ist federelastisch.

Dies ist vorteilhafterweise dadurch realisiert, dass das drahtförmige Element zumindest im Bereich der Schleife aus einem federelastischen Material, erfindungsgemäß aus Federstahl, gebildet ist. Eine gewisse Federelastizität ergibt sich jedoch bereits durch die gebogene Konfiguration des drahtförmigen Elementes als Schleife und kann durch die zuvor genannte Materialwahl noch verstärkt werden. Die Federelastizität der Schleife kann vorteilhafterweise das Verschwenken des zumindest einen beweglichen Maulteils unterstützen, bspw. entweder die Öffnungebewagung oder die Schließbewegung des zumindest einen beweglichen Maulteils.

In einer bevorzugten Ausgestaltung erstreckt sich die Schleife über zumindest etwa einen Halbkreis.

Diese Maßnahme hat den Vorteil, dass die Gesamtlänge des drahtförmigen Elements gegenüber einem geradlinig geführten drahtförmigen Element nur geringfügig erhöht ist, und andererseits bei medizinischen Instrumenten, bei denen das zumindest eine bewegliche Maulteil nur einen geringen maximalen Schwenkwinkel durchläuft, ausreichend ist, um die erfindungsgemäße Wirkung wie oben beschrieben herbeizuführen.

Besonders bevorzugt ist es, wenn sich die Schleife über zumindest einen Vollkreis erstreckt.

Bei dieser Ausgestaltung weist das drahtförmige Element zumindest eine vollständige Windung auf. Das proximale und das distale Ende des drahtförmigen Elements bilden bei dieser Ausgestaltung eine Art Schenkelfeder wie bei einer Wäscheklammer, wobei für die Zwecke der vorliegenden Erfindung eine Windung als Spiral- oder als Schraubenfeder ausreichend ist, es können jedoch auch mehr Windungen vorgesehen sein.

In einer weiter bevorzugten Ausgestaltung ist die Schleife um die Schwenkachse herum angeordnet.

Während es im Rahmen der vorliegenden Erfindung auch vorgesehen sein kann, die Schleife distalseitig oder proximalseitig von der Schwenkachse anzuordnen, hat die vorstehend genannte Maßnahme den Vorteil, dass die Schleife an dieser Stelle ohne zusätzlichen Platzbedarf angeordnet werden kann. Der weitere Vorteil dieser Maßnahme besteht darin, dass das drahtförmige Element im Bereich der Schwenkachse am stärksten auf Biegung beansprucht wird, und somit der durch die Schleife bewirkte Längenausgleich am besten zum Tragen kommt.

In einer weiteren bevorzugten Ausgestaltung ist ein proximales Ende des drahtförmigen Elements am Rohrschaft festgelegt und leitend mit diesem verbunden.

Diese Maßnahme hat den Vorteil, dass der Rohrschaft wie bei dem bekannten Instrument als Stromzuleitung genutzt werden kann. Das proximale Ende des drahtförmigen Elements kann dabei im Bereich des distalen Endes des Rohrschafts festgelegt werden, was den weiteren Vorteil hat, dass das drahtförmige Element nicht vollständig durch den Rohrschaft hindurch nach proximal geführt werden muss, was den Zusammenbau des erfindungsgemäßen Instruments vereinfacht.

In einer weiteren bevorzugten Ausgestaltung ist am distalen Ende des Rohrschafts ein zweites bewegliches Maulteil angeordnet, das um die Schwenkachse verschwenkbar ist und eine zweite Elektrode aufweist, und ein axial bewegliches Kraftübertragungselement eines Betätigungsmechanismus zum Bewegen der beiden Maulteile dient als Stromzuleitung für die zweite Elektrode.

Bei dieser Ausgestaltung kommen die vorteilhaften Wirkungen der vorliegenden Erfindung besonders zum Tragen, da für beide Elektroden der beiden beweglichen Maulteile eine Stromzuführung vorhanden ist, bei denen eine Biegebeanspruchung und damit eine Bruchgefährdung vermieden wird.

In diesem Zusammenhang ist es bevorzugt, wenn die beiden Maulteile im Bereich der Schwenkachse gegeneinander isoliert sind.

Eine solche Isolierung kann wie bei dem aus dem Dokument WO-A-01/15614 bekannten Instrument vorgesehen sein, in dem die Maulteile, oder zumindest eines der beiden Maulteile, im Bereich der Schwenkachse einen Grundkörper aus einem elektrisch isolierenden Material aufweist. Dabei kann dann ein Niet oder Zapfen, der die Schwenkachse bildet, metallisch und damit besonders stabil ausgeführt werden, da über den isolierenden Grundkörper eine elektrische Trennung der beiden Maulteile voneinander bewerkstelligt ist.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese nachfolgend näher beschrieben. Es zeigen:
- Fig. 1: ein medizinisches Instrument für die Elektrochirurgie in Seitenansicht; und
- Fig. 2: das distale Ende des Instruments in Fig. 1 in vergrö- ßertem Maßstab, teilweise im Längsschnitt.

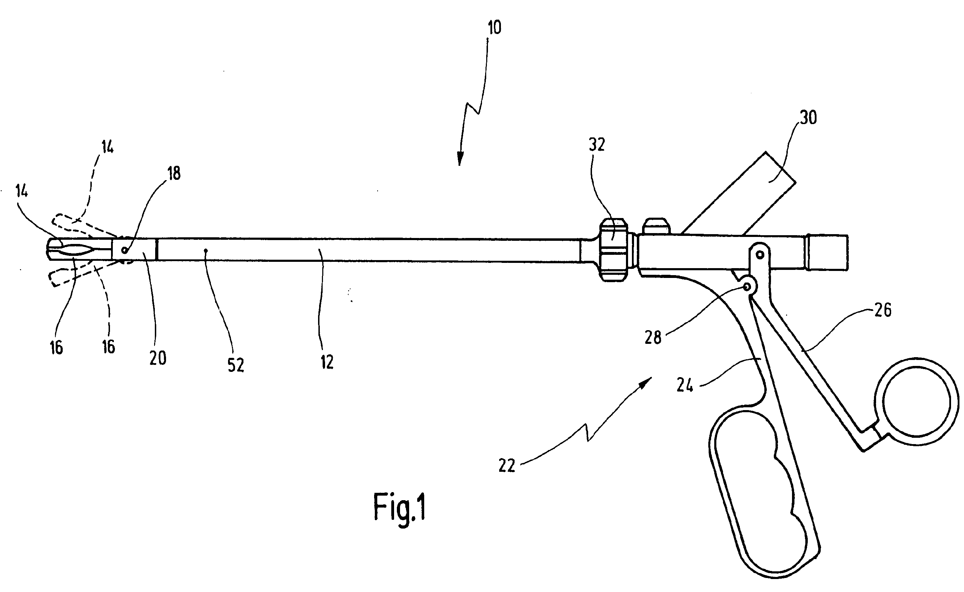

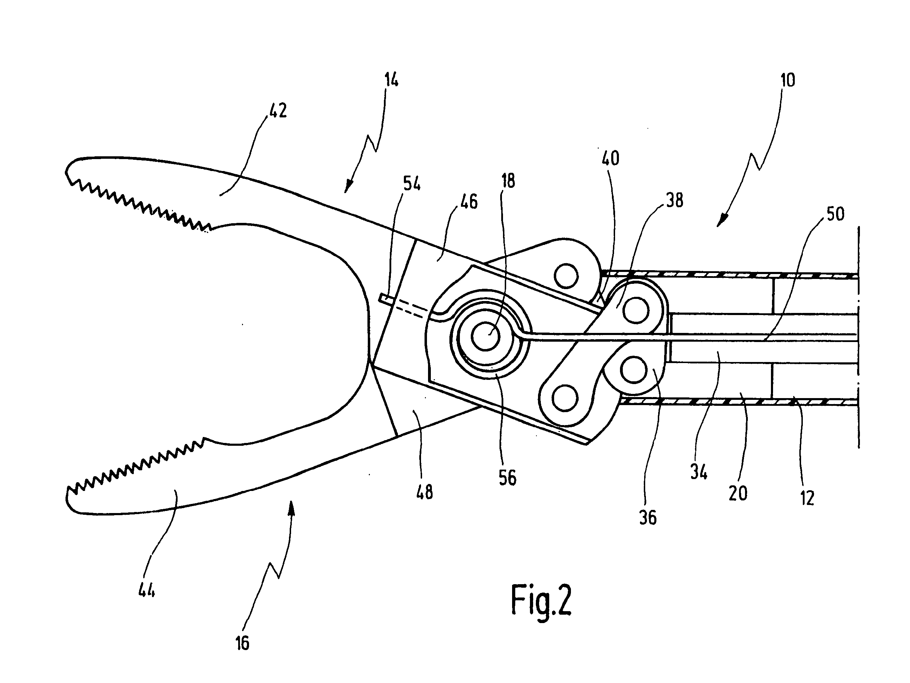

In Fig. 1 ist ein mit dem allgemeinen Bezugszeichen 10 versehenes medizinisches Instrument für die Elektrochirurgie dargestellt. In Fig. 2 sind weitere Einzelheiten des Instruments 10 näher dargestellt.

Das Instrument 10 wird im Rahmen der minimal-invasiven Chirurgie zur Behandlung von Gewebe im menschlichen oder tierischen Körper zur Präparation mittels Hochfrequenzstrom verwendet.

Das Instrument 10 ist in dem gezeigten Ausführungsbeispiel ein Fassinstrument, genauer eine Fasszange, mit der Gewebe gefasst und koaguliert werden kann. Das Instrument 10 ist ferner ein bipolares Instrument, wie hiernach noch näher erläutert wird.

Das Instrument 10 weist einen langerstreckten Rohrschaft 12 auf. Der Rohrschaft 12 ist ein als Stromleiter ausgebildetes metallisches Rohr, das von einer isolierenden Umhüllung umgeben ist.

Am distalen Ende des Rohrschafts 12 sind ein erstes Maulteil 14 und ein zweites Maulteil 16 angeordnet.

Das erste Maulteil 14 und das zweite Maulteil 16 sind beide relativ zum Rohrschaft 12 um eine Schwenkachse 18 beweglich, das heißt verschwenkbar. In Fig. 1 sind die Maulteile 14 und 16 bezüglich ihrer Schließlage mit durchgezogenen Linien, und bezüglich ihrer Offenstellung mit unterbrochenen Linien dargestellt.

Die Maulteile 14 und 16 sind im Bereich der Schwenkachse 18 an einem Isolatorstück 20 aus Kunststoff oder Keramik befestigt, das seinerseits mit dem Rohrschaft 12 verbunden ist.

Am proximalen Ende des Rohrschafts 12 ist eine Handhabe 22 angeordnet, die zwei Griffteile 24 und 26 aufweist. Die Griffteile 24 und 26 sind relativ zueinander um eine Schwenkachse 28 verschwenkbar gelenkig miteinander verbunden, wobei die Relativbewegung der Griffteile 24 und 26 dem Öffnen und Schließen der Maulteile 14 und 16 dient.

Des Weiteren ist an der Handhabe 22 ein Anschluss 30 zum Anschließen des Instruments 10 an einen nicht dargestellten Hochfrequenzstromgenerator angeordnet.

Der Rohrschaft 12 ist ferner um seine Längsachse verdrehbar, wozu zur Betätigung ein Drehgriff 32 am distalen Ende der Handhabe 22 angeordnet ist.

Durch den Rohrschaft 12 verläuft von der Handhabe 22 zu den Maulteilen 14 und 16 ein axial bewegliches Kraftübertragungselement 34, das in Fig. 2 im Bereich seines distalen Endes dargestellt ist, und dessen proximales Ende mit einem der beiden Griffteile 24 bzw. 26 kraftschlüssig verbunden ist. Das Kraftübertragungselement 34 ist über einen Gelenkhebelmechanismus 36, 38, 40 mit beiden Maulteilen 14 und 16 verbunden. Eine axiale Bewegung des Kraftübertragungselements 34 dient dem Öffnen und Schließen, das heißt dem Verschwenken der Maulteile 14 und 16 um die Schwenkachse 18.

Die Schwenkachse 18 ist bspw. durch einen quer zur Längsrichtung des Schafts 12 verlaufenden Niet oder Achszapfen gebildet.

Weiter mit Bezug auf Fig. 2 werden nun weitere Einzelheiten des Instruments 10 im Bereich der Maulteile 14 und 16 beschrieben.

Das Maulteil 14 weist eine Elektrode 42 auf, und das Maulteil 16 weist eine Elektrode 44 auf, wobei die Elektroden 42 und 44 das distale Ende der Maulteile 14 bzw. 16 bilden.

Im proximalen Bereich weist das Maulteil 14 einen Grundkörper 46 aus isolierendem Material und das Maulteil 16 einen Grundkörper 48 ebenfalls aus isolierendem Material auf. Auf diese Weise sind die Maulteile 14 und 16 in ihrem proximalen Bereich elektrisch gegeneinander isoliert.

Als Stromzuleitung für die Elektrode 42 des Maulteils 14 dient ein drahtförmiges Element 50, dessen proximales Ende mit dem als Stromleiter ausgebildeten Rohrschaft 12 verbunden ist, bspw. im Bereich der in Fig. 1 mit dem Bezugszeichen 52 versehenen Stelle. Über den Rohrschaft 12 und das drahtförmige Element 50 kann die Elektrode 42 somit an einen ersten Pol des Hochfrequenzstromgenerators angeschlossen werden.

Ein distales Ende 54 des drahtförmigen Elements 50 ist mit der Elektrode 42 stromleitend verbunden.

Im Bereich der Schwenkachse 18 ist das drahtförmige Element 50 als Schleife 56 ausgebildet, die um die Schwenkachse 18 herum angeordnet ist.

Die Schleife 56 ist im vorliegenden Ausführungsbeispiel dadurch gebildet, dass das drahtförmige Element 50 etwa eineinhalb Mal um die Schwenkachse 18 herumgelegt ist, und somit zumindest eine Windung aufweist. Die Schleife 56 bewirkt beim Öffnen und Schließen des Maulteils 14 einen Längenausgleich, wobei das drahtförmige Element 50 dabei wesentlich geringer auf Biegung oder Streckung beansprucht wird.

Während in dem gezeigten Ausführungsbeispiel die Schleife 56 sich somit über zumindest einen Vollkreis erstreckt, kann die Schleife 56 jedoch auch sich nur über zumindest etwa einen Halbkreis erstrecken, wenn dies den Anforderungen genügt.

Die Schleife 56 ist insbesondere federelastisch, wozu das drahtförmige Element 50 zumindest im Bereich der Schleife 56 aus einem federelastischen stromleitenden Material, insbesondere aus Federstahl, gebildet ist.

Die Elektrode 44 des zweiten Maulteils 16 wird demgegenüber über das Kraftübertragungselement 34, das entsprechend stromleitend ausgebildet ist, an den anderen Pol des Hochfrequenzgenerators angeschlossen, wobei die Stromübertragung vom Kraftübertragungselement 34 über den Gelenkhebelmechanismus 36, 40 erfolgt, wie dies in dem Dokument WO-A-01/15614 beschrieben ist, auf deren Offenbarung diesbezüglich ausdrücklich Bezug genommen wird.

## Patentansprüche

1. Medizinisches Instrument für die Elektrochirurgie, mit einem Rohrschaft (12), mit zumindest einem um eine Schwenkachse (18) relativ zum Rohrschaft (12) beweglichen Maulteil (14) am distalen Ende des Rohrschafts (12), wobei das zumindest eine bewegliche Maulteil (14) eine Elektrode (42) aufweist, und mit einer zu der Elektrode (42) des zumindest einen beweglichen Maulteils (14) führenden Stromzuleitung, die als drahtförmiges Element (50) aus stromleitendem Material ausgebildet ist, dessen distales Ende (54) mit der Elektrode (42) verbunden ist, wobei das drahtförmige Element (50) im Bereich der Schwenkachse (18) als Schleife (56) ausgebildet ist, und wobei das drahtförmige Element (50) zumindest im Bereich der Schleife (56) aus einem federelastischen Material gebildet ist, **dadurch gekennzeichnet, dass** das drahtförmige Element (50) zumindest im Bereich der Schleife (56) aus Federstahl gebildet ist.

2. Instrument nach Anspruch 1, **dadurch gekennzeichnet, dass** sich die Schleife (56) über zumindest etwa einen Halbkreis erstreckt.

3. Instrument nach Anspruch 1 oder 2, **dadurch gekennzeichnet, dass** sich die Schleife (56) über zumindest einen Vollkreis erstreckt.

4. Instrument nach einem der Ansprüche 1 bis 3, **dadurch gekennzeichnet, dass** die Schleife (56) um die Schwenkachse (18) herum angeordnet ist.

5. Instrument nach einem der Ansprüche 1 bis 4, **dadurch gekennzeichnet, dass** ein proximales Ende des drahtförmigen Elements (50) am Rohrschaft (12) festgelegt und leitend mit diesem verbunden ist.

6. Instrument nach einem der Ansprüche 1 bis 5, **dadurch gekennzeichnet, dass** am distalen Ende des Rohrschafts (12) ein zweites bewegliches Maulteil (16) angeordnet ist, das um die Schwenkachse (18) verschwenkbar ist und eine zweite Elektrode (44) aufweist, und dass ein axial bewegliches Kraftübertragungselement (34) eines Betätigungsmechanismus zum Bewegen der beiden Maulteile (14, 16) als Stromzuleitung für die zweite Elektrode (44) dient.

7. Instrument nach Anspruch 6, **dadurch gekennzeichnet, dass** die beiden Maulteile (14, 16) im Bereich der Schwenkachse (18) gegeneinander isoliert sind.

## Claims

1. A medical instrument for electrosurgery, comprising a tubular shaft (12), at least one jaw part (14) arranged at the distal end of the tubular shaft (12) and movable relative to the tubular shaft (12) about a pivot axis (18), said at least one movable jaw part (14) having an electrode (42), and comprising a current supply line which leads to the electrode (42) of the at least one movable jaw part (14) and which is designed as a wire-shaped element (50) made of an electrically conducting material, whose distal end (54) is connected to the electrode (42), wherein the wire-shaped element (50) is configured as a loop (56) in the area of the pivot axis (18), and wherein the wire-shaped element (50), at least in the area of the loop (56), is made of a resilient material, **characterized in that** the wire-shaped element (50), at least in the area of the loop (56), is made of a spring steel.

2. The instrument of Claim 1, **characterized in that** the loop (56) extends over at least approximately half a circle.

3. The instrument of Claim 1 or 2, **characterized in that** the loop (56) extends over at least one full circle.

4. The instrument of any one of Claims 1 through 3, **characterized in that** the loop (56) is arranged around the pivot axis (18).

5. The instrument of any one of Claims 1 through 4, **characterized in that** a proximal end of the wire-shaped element (50) is fixed on the tubular shaft (12) and connected conductively to the latter.

6. The instrument of any one of Claims 1 through 5, **characterized in that** the distal end of the tubular shaft (12) is provided with a second movable jaw part (16) which is pivotable about the pivot axis (18) and has a second electrode (44), and **in that** an axially movable force transmission element (34) of an actuating mechanism for moving the two jaw parts (14, 16) serves as current supply line for the second electrode (44).

7. The instrument of Claim 6, **characterized in that** the two jaw parts (14, 16) are insulated from one another in the area of the pivot axis (18).

## Revendications

1. Instrument médical pour l'électrochirurgie, avec au moins une tige tubulaire (12), ayant, à l'extrémité distale de la tige tubulaire (12), au moins une partie de mâchoire (14) mobile autour d'un axe de pivotement (18) par rapport à la tige tubulaire (12), sachant que la au moins une partie de mâchoire (14) mobile présente une électrode (42), et avec une conduite d'amenée de courant vers l'électrode (42) de la au moins une partie de mâchoire mobile (14), qui est constituée d'une élément (50) en matériau conducteur sous la forme d'un fil, dont l'extrémité distale (54) est reliée à l'électrode (42), sachant que l'élément sous forme de fil (50) est réalisé dans le domaine de l'axe de pivotement (18) en tant que boucle (56), et que l'élément sous forme de fil (50) est constitué, au moins dans le domaine de la boucle (56), d'un matériau élastique, **caractérisé en ce que** l'élément sous forme de fil (50) est réalisé, au moins dans le domaine de la boucle (56), à l'aide d'acier à ressort.

2. Instrument selon la revendication 1, **caractérisé en ce que** la boucle (56) se prolonge sur au moins en gros un demi-cercle.

3. Instrument selon la revendication 1 ou 2, **caractérisé en ce que** la boucle (56) se prolonge sur au moins un cercle complet.

4. Instrument selon l'une quelconque des revendications 1 à 3, **caractérisé en ce que** la boucle (56) est disposée autour de l'axe de pivotement (18).

5. Instrument selon l'une quelconque des revendications 1 à 4, **caractérisé en ce que** l'extrémité proximale de l'élément sous forme de fil (50) est fixée à la tige tubulaire (12) et reliée à cette dernière d'une manière conductrice.

6. Instrument selon l'une quelconque des revendications 1 à 5, **caractérisé en ce que** l'on dispose à l'extrémité distale de la tige tubulaire (12) une seconde partie de mâchoire mobile (16), qui peut pivoter autour de l'axe de pivotement (18) et qui présente une seconde électrode (44) et **en ce qu'**un élément de transfert de forces mobile en direction axiale (34) d'un mécanisme d'actionnement destiné à faire bouger les deux parties de mâchoire (14, 16) fait office de conducteur de courant pour la seconde électrode (44).

7. Instrument selon la revendication 6, **caractérisé en ce que** les deux parties de mâchoire (14, 16) sont isolées l'une par rapport à l'autre dans le domaine de l'axe de pivotement (18).
